(19) Europäisches Patentamt
European Patent Office
Office européen des brevets

(11) Veröffentlichungsnummer: **0 175 649**
**B1**

(12) # EUROPÄISCHE PATENTSCHRIFT

(45) Veröffentlichungstag der Patentschrift: **02.01.91**

(51) Int. Cl.[5]: **C 07 C 267/00,**
C 07 D 213/62, A 01 N 47/40

(21) Anmeldenummer: **85810416.9**

(22) Anmeldetag: **13.09.85**

(54) **Substituierte Carbodiimide.**

(30) Priorität: **19.09.84 CH 4479/84**
**15.08.85 CH 3526/85**

(43) Veröffentlichungstag der Anmeldung:
**26.03.86 Patentblatt 86/13**

(45) Bekanntmachung des Hinweises auf die Patenterteilung:
**02.01.91 Patentblatt 91/01**

(84) Benannte Vertragsstaaten:
**AT BE CH DE FR IT LI NL**

(56) Entgegenhaltungen:
**DE-B-1 121 402**
**GB-A-1 476 086**
**SYNTHESIS, Nr. 12, Dezember 1977, Seiten 889,890, Stuttgart, DE; T. FUJINAMI et al.: "Preparation of carbodiimides by the reaction of dimetallothioureas with sulfur dioxide"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Böger, Manfred**
**Wilhelm Glockstrasse 14**
**D-7858 Weil am Rhein 5 (DE)**
Erfinder: **Drabek, Jozef, Dr.**
**Benkenstrasse 12**
**CH-4104 Oberwil (CH)**

**EP 0 175 649 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neuartige substituierte N-Phenyl-N'-alkyl-carbodiimide, Verfahren zu ihrer Herstellung und ihre Verwendung in der Schädlingsbekämpfung.

Diese erfindungsgemässen Verbindungen haben die Formel I

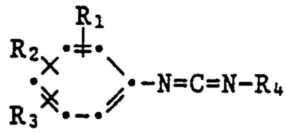

(I),

worin

$R_1$ Wasserstoff, Halogen, $C_1$—$C_4$-Alkyl, Methoxy, Aethoxy, Trifluormethoxy oder $C_1$—$C_3$-Alkylthio;
$R_2$ Wasserstoff, Halogen, $C_1$—$C_4$-Alkyl, Methoxy oder Aethoxy,
$R_3$ Wasserstoff, $C_1$—$C_4$-Alkyl, Halogen, Phenoxy, Phenylthio, ein- oder zweifach mit Halogen und/oder Trifluormethyl substituiertes Phenoxy, Pyridyloxy oder ein- oder zweifach mit Halogen oder Trifluormethyl substituiertes Pyridyloxy; und
$R_4$ $C_1$—$C_8$-Alkyl, Alkoxyalkyl mit insgesamt 2 bis 7 C-Atomen, $C_3$—$C_5$-Cycloalkyl, $C_3$—$C_6$-Cycloalkylmethyl oder Methyl-($C_3$—$C_6$)-Cycloalkyl;

bedeuten, wobei mindestens zwei der Reste $R_1$, $R_2$ oder $R_3$ eine von Wasserstoff abweichende Bedeutung besitzen, $R_3$ in 4-Stellung und $R_1$ und $R_2$ unabhängig voneinander in 2- bzw. 6-Stellung am Phenylrest stehen.

Die bei $R_1$ bis $R_4$ in Frage kommenden Alkyl- und Alkoxygruppen und -Substituenten können geradkettig oder verzweigt sein. Beispiele solcher Gruppen sind dementsprechend z.B. Methyl, Methoxy, Aethyl, Propyl, Isopropyl, n-, i-, sek.-, tert.-Butyl, n-Pentyl, n-Hexyl, n-Heptyl, n-Octyl und deren Isomere.

Unter Halogen im Rahmen der vorliegenden Erfindung ist vorzugsweise F, Cl und Br zu verstehen, insbesondere F und Cl.

Wegen ihrer Wirkung als Schädlingsbekämpfungsmittel bevorzugt sind Verbindungen der Formel I, die dadurch gekennzeichnet sind, dass

$R_1$ Wasserstoff, Fluor, Chlor, $C_2$—$C_4$-Alkyl oder Methoxy;
$R_2$ Wasserstoff, Chlor, $C_3$—$C_4$-Alkyl oder Methoxy;
$R_3$ Methyl, Aethyl, Isopropyl, Chlor, Phenoxy, ein- oder zweifach mit Chlor und/oder Trifluormethyl substituiertes Phenoxy, Pyridyloxy oder ein- oder zweifach mit Chlor und/oder Trifluormethyl substituiertes Pyridyloxy; und
$R_4$ $C_3$—$C_8$-Alkyl, $C_1$—$C_4$-Alkoxy-($C_1$—$C_3$)-Alkyl oder $C_3$—$C_5$-Cycloalkyl bedeuten.

Hervorzuheben sind insbesondere Verbindungen der Formel I, worin $R_4$ Isopropyl oder tert. Butyl bedeutet.

Die Verbindungen der Formel I können nach an sich bekannten Verfahren hergestellt werden. So kann man z.B. eine Verbindung der Formel I erhalten, indem man aus einer Verbindung der Formel II

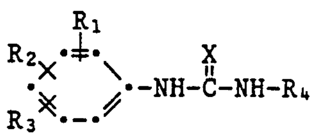

(II),

worin $R_1$ bis $R_4$ die vorstehend angegebenen Bedeutungen haben und X für Sauerstoff oder Schwefel steht, Wasser oder Schwefelwasserstoff abspaltet. Derartige Abspaltungs-Reaktionen können nach literaturbekannten Arbeitsweisen z.B. mit Hilfe von HgO, bestimmten Pyridiniumsalzen, Chloressigsäureestern, Cyanursäurechlorid, p-Toluolsulfochlorid oder bestimmten Phosphorsäureester-Derivaten durchgeführt werden [T. Shibanuma, Chemistry Letters (*1977*), p. 575—6; S. Kim, Tetrahedron Letters (*1985*), p. 1661—1664; W. Weith, B.*6* (1873) 1398; G. Amiard, Bull. Soc. chim. *1956*, 1360].

Das vorerwähnte Verfahren kann üblicherweise unter normalen Druck und in Gegenwart eines vorzugsweise aprotischen organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; sowie Ketone, z.B. Aceton, Methyläthylketon, Methylisopropylketon und Methylisobutylketon. Das Verfahren wird im allgemeinen bei einer Temperatur von −5 bis +150°C, insbesondere zwischen 10 und 50°C, z.B. bei Raumtemperatur, durchgeführt.

Man kann die Carbodiimide der Formel I auch in an sich bekannter Weise herstellen durch Umsetzung

von entsprechend substituierten Isocyaniddichloriden der Formel III mit einem Salz des jeweils erwünschten primären Amins der Formel IV (vgl. US—PS—3.231.610)

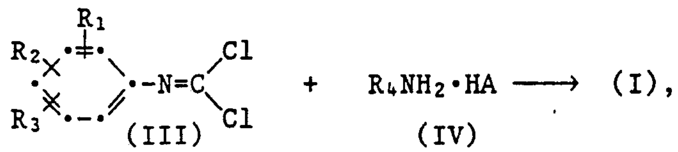

wobei in den Formeln III und IV die Reste $R_1$ bis $R_4$ die vorstehend angegebenen Bedeutungen haben und A ein Anion, z.B. $Cl^{\ominus}$, bedeutet.

Bei dieser Umsetzung sind als primäre Aminsalze z.B. die Hydrohalogenide geeignet. Die Umsetzung wird vorzugsweise ausgeführt in Gegenwart eines inerten organischen Lösungsmittels von relativ hohem Siedepunkt, wie z.B. chlorierte Benzole, Nitrobenzol, Dimethylacetamid oder Tetramethylensulfon. Weiterhin können als Lösungsmittel z.b. die folgenden in Betracht kommen: hochsiedende aliphatische, cycloaliphatische und aromatische Kohlenwasserstoffe, wie p-Chlorbrombenzol, 1-Chlornaphthalin oder halogenierte Xylole. Bevorzugt wird im allgemeinen eine Umsetzungstemperatur von 80 bis 200°C.

Die Ausgangsstoffe der Formeln II, III und IV sind bekannt und können nach bekannten Arbeitsweisen erhalten werden (vgl. BE—PS—863.078, DE—Patentanmeldung 1.094.737 und US—PS—3.932.507).

Es ist bereits aus der deutschen Offenlegungsschrift 2 553 270 bekannt, N,N'-diphenylcarbodiimide als Akarizide, insbesondere als Ektoparasitizide, zu verwenden. Der Einsatz von substituierten N-Benzyl-N'-alkyl-carbodiimiden als Insektizide wird in der japanischen Patentveröffentlichung 5.0069.226 beschrieben. N-Alkyl-N'-(2-phenyl-2-propyl)-carbodiimide mit insektizider Wirkung sind in der britischen Patentschrift 1.476.086 aufgeführt. Auch die USA—Patentschrift 3.231.610 bezieht sich auf substituierte Carbodiimide, die herbizide und insektizide Eigenschaften besitzen sollen. Von der in dieser USA—Patentschrift angegebenen allgemeinen Formel werden neben N,N'-Dialkylcarbodiimiden und N,N'-Diphenylcarbodiimiden auch bestimmte N-Phenyl-N'-alkylcarbodiimide umfasst; spezifisch offenbart werden in der genannten USA—Patentschrift jedoch nur Chlor-oder Nitro-substituierte N,N'-Diphenylcarbodiimide, nicht jedoch N-Phenyl-N'-alkylcarbodiimide. Ferner werden in der deutschen Auslegeschrift 1121402 bestimmte substituierte N-Phenyl-N'-alkylcarbodiimide als Insektizide offenbart, wie das N-(2-Methylphenyl)-N'-isopropylcarbodiimid.

Demgegenüber stellen die gemäss vorliegender Erfindung vorgeschlagenen substituierten N-Phenyl-N'-alkylcarbodiimide neuartige Verbindungen dar, die überraschenderweise bei guter Pflanzenverträglichkeit und geringer Warmblütertoxizität eine ausgeprägte Wirksamkeit zur Bekämpfung von Pflanzen und Tiere befallenden Insekten und Vertretern der Ordnung Akarina besitzen. Die erfindungsgemässen Verbindungen der Formel I besitzen hohe insektizide Wirksamkeit und sind z.B. dem aus der deutschen Auslegeschrift 1121402 bekannten N-(2-Methylphenyl)-N'-isopropylcarbodiimide als Insektizide überlegen.

Die Verbindungen der Formel I eignen sich insbesondere zur bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina, insbesondere von pflanzenschädigenden Akarinen, wie z.B. Spinnmilben.

Die gute pestizide Wirkung der erfindungsgemässen Verbindungen der Formel I entspricht einer Abtötungsrate (Mortalität) von mindestens 50—60% der erwähnten Schädlinge.

Neben ihrer sehr günstigen Wirkung gegenüber Fliegen, wie z.b. Musca domestica und Mückenlarven, eignen sich Verbindungen der Formel I vor allem zur Bekämpfung von pflanzenschädigenden Frassinsekten in Zier- und Nutzpflanzungen, insbesondere in Baumwollkulturen (z.B. Spodoptera littoralis und Heliothis virescens) sowie in Gemüsekulturen (z.B. Leptinotarsa decemlineata und Pieris brassicae). Hervorzuheben ist auch dei larvizide und ovizide .Wirkung von Verbindungen der Formel I. Werden Verbindungen der Formel von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I sind weiterhin zur Bekämpfung von Ektoparasiten, z.B. von Lucilia sericata, sowie von Zecken an Haus- und Nutztieren geeignet, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und wiesen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethyklenoxid-Addukten. Die sulfonierten Benzimidazolderivate enthalten vorzugsweise 2 Sulfonsäuregruppen und einen Fettsäurerest mit etwa 8—22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4—14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95%, Wirkstoff der Formel I oder Kombinationen dieser Wirkstoffe mit anden Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effeke enthalten.

Beispiel 1

Herstellung von N-[2,6-Dimethyl-4-(3,5-dichlor-2-pyridyloxy)-phenyl]-N'-tert.butylcarbodiimid

16,6 g N-[2,6-Dimethyl-4-(3,5-dichlor-2-pyridyloxy)-phenyl]-N'-tert.butylthioharnstoff und 12,8 g 2-Chlor-1-methyl-pyridiniumjodid werden in 150 ml Acetonitril vorgelegt. Bei Raumtemperatur wird unter Rühren eine Lösung von 8,4 g Triäthylamin in 80 ml Acetonitril zugetropft, und anschliessend wird während 2 Stunden bei 80°C gerührt.

Das Reaktionsgemisch wird dann am Rotationsverdampfer bei 50°C eingedampft. Der Rückstand wird mit 150 ml Hexan versetzt und filtriert. Die Hexanphase wird dreimal mit 30 ml kaltem Wasser gewaschen, über Natriumsulfat getrocknet, filtriert und eingedampft. Man erhält so die Titelverbindung der Formel

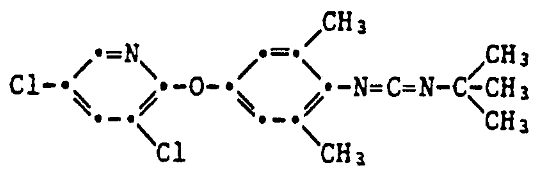

als klares blassgelbes Oel, das beim Stehenlassen auskristallisiert und einen Schmelzpunkt von 69—71°C aufweist (Verbindung Nr. 1).

Entsprechend den vorstehend beschriebenen Arbeitsweisen werden auch die folgenden Verbindungen der Formel I erhalten:

| Verbindung Nr. | $R_1$ | $R_2$ | $R_3$ | $R_4$ | physik. Daten |
|---|---|---|---|---|---|
| 2 | 2-$CH_3$ | 6-$CH_3$ | 4-O- 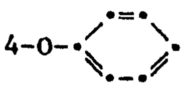 | -$C_4H_9$(t) | $n_{20}^{D}$=1,5764 |
| 3 | 2-$C_3H_7$(i) | 6-$C_3H_7$(i) | H | -$C_4H_9$(t) | weisse, sich bei Raumtemperatur verflüssigende viskose Masse |
| 4 | 2-$C_3H_7$(i) | 6-$C_3H_7$(i) | 4-O- 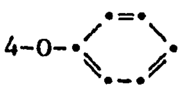 | -$C_4H_9$(t) | Smp. 45-46°C |
| 5 | 2-Cl | H | 4-Cl | -$C_4H_9$(t) | $n_{20}^{D}$=1,5895 |
| 6 | 6-$C_4H_9$(s) | 2-$C_2H_5$ | 4-O- 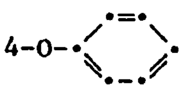 | -$C_4H_9$(t) | $n_{22}^{D}$=1,5575 |
| 7 | 2-$C_3H_7$(i) | H | 4-O- 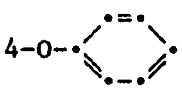 -$CF_3$ | -$C_4H_9$(t) | $n_{20}^{D}$=1,5300 |
| 8 | 2-$CH_3$ | 6-$CH_3$ | 4-O- 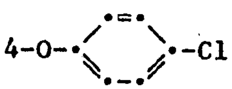 -Cl | -$C_4H_9$(t) | $n_{20}^{D}$=1,5815 |
| 9 | 2-$CH_3$ | 6-$CH_3$ | 4-O- 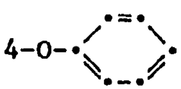 -$CF_3$ | -$C_4H_9$(t) | $n_{20}^{D}$=1,5374 |
| 10 | 2-$CH_3$ | 6-$CH_3$ | 4-O- 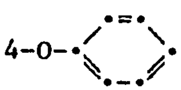 | -$CH_2$-$C_4H_9$(t) | $n_{20}^{D}$=1,5720 |
| 11 | 2-$CH_3$ | 6-$CH_3$ | 4-O- 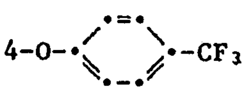 -$CF_3$ | 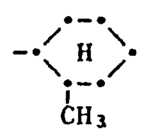 | $n_{20}^{D}$=1,5505 |
| 12 | 2-$CH_3$ | H | 4-Cl | -$CH_3$ | $n_{20}^{D}$ = 1,6230 |
| 13 | 2-$CH_3$ | 6-$CH_3$ | 4-O- 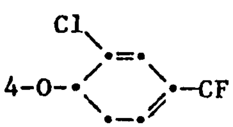 -$CF_3$ | -$C_4H_9$(t) | $n_{20}^{D}$=1,5460 |

| Verbindung Nr. | $R_1$ | $R_2$ | $R_3$ | $R_4$ | physik. Daten |
|---|---|---|---|---|---|
| 14 | $2-CH_3$ | $6-CH_3$ | 4-O- 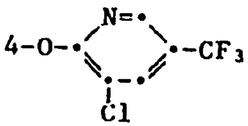 | $-C_3H_7(i)$ | Smp. 50-52°C |
| 15 | $2-CH_3$ | $6-CH_3$ | 4-O- 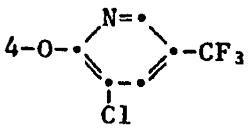 | $-C_4H_9(t)$ | Smp. 65- 68°C |
| 16 | $6-CH_3$ | $2-CH_3$ | 4-O- 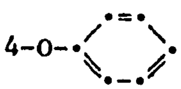 | 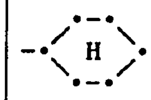 | $n_{27}^{D}=1,5905$ |
| 17 | $6-OCH_3$ | $2-OCH_3$ | H | $-C_4H_9(t)$ | Smp. 61-64°C |
| 18 | H | $2-CH_3$ | 4-O- 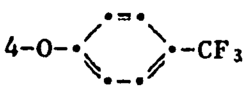 | $C_4H_9(t)$ | $n_{21}^{D}=1,5350$ |
| 19 | $2-C_4H_9(s)$ | $6-C_2H_5$ | 4-O- 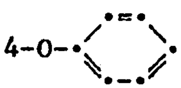 | 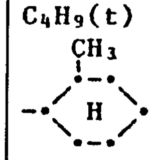 | $n_{21}^{D}=1,5672$ |
| 20 | $2-OCH_3$ | $6-OCH_3$ | H | 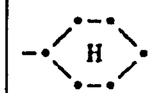 | Smp. 57-60°C |
| 21 | $2-CH_3$ | $6-CH_3$ | 4-O- 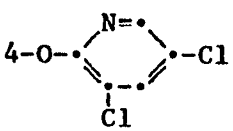 | $-(CH_2)_3-O-(CH_2)_3-CH_3$ | $n_{21}^{D}=1,5726$ |
| 22 | $2-CH_3$ | $6-CH_3$ | 4-O- 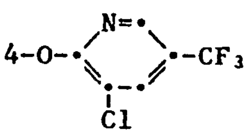 | $-(CH_2)_3-O-(CH_2)_3-CH_3$ | $n_{21}^{D}=1,5355$ |
| 23 | $2-C_3H_7(i)$ | $6-C_3H_7(i)$ | H | 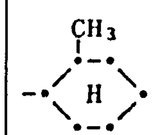 | $n_{21}^{D}=1,5388$ |
| 24 | $2-C_4H_9(s)$ | $6-C_2H_5$ | 4-O- 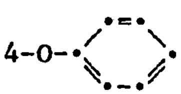 | 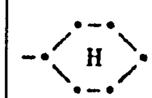 | $n_{21}^{D}=1,5721$ |

| Verbindung Nr. | $R_1$ | $R_2$ | $R_3$ | $R_4$ | physik. Daten |
|---|---|---|---|---|---|
| 25 | $2-C_3H_7(i)$ | $6-C_3H_7(i)$ | H | 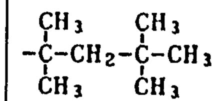 | $n_{21}^{D}=1,5177$ |
| 26 | $2-C_3H_7(i)$ | $6-C_3H_7(i)$ | H | 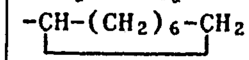 | $n_{21}^{D}=1,5473$ |
| 27 | $2-CH_3$ | H | $4-CH_3$ | $-C_4H_9(t)$ | $n_{21}^{D}=1,5340$ |
| 28 | $2-CH_3$ | $6-CH_3$ | H | $-C_3H_7(i)$ | $n_{23}^{D}=1,5459$ |
| 29 | $2-CH_3$ | $6-CH_3$ | H | $-C_4H_9(t)$ | $n_{23}^{D}=1,5354$ |
| 30 | $2-CH_3$ | $6-CH_3$ | H | $-C_4H_9(s)$ | $n_{23}^{D}=1,5424$ |
| 31 | 2-Cl | H | 4-Cl | $-C_4H_9(t)$ | $n_{22}^{D}=1,5650$ |
| 32 | 2-Cl | H | 4-Cl | $-C_3H_7(i)$ | $n_{22}^{D}=1,5826$ |
| 33 | $2-C_2H_5$ | $6-C_4H_9(s)$ | H | $-C_4H_9(t)$ | $n_{23}^{D}=1,5230$ |
| 34 | $6-C_3H_7(i)$ | $2-C_2H_5$ | H | $-C_3H_7(i)$ | $n_{25}^{D}=1,5300$ |
| 35 | $2-C_3H_7(i)$ | $6-CH_3$ | H | $-C_4H_9(t)$ | $n_{23}^{D}=1,5261$ |
| 36 | $2-C_3H_7(i)$ | $6-C_2H_5$ | H | $-C_4H_9(t)$ | $n_{23}^{D}=1,5256$ |
| 37 | $2-C_3H_7(i)$ | $6-C_3H_7(i)$ | H | $-C_3H_7(i)$ | $n_{23}^{D}=1,5293$ |
| 38 | $2-CH_3$ | H | $4-CH_3$ | $-C_3H_7(i)$ | $n_{22}^{D}=1,5450$ |
| 39 | $2-C_2H_5$ | $6-C_4H_9(s)$ | H | 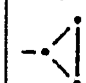 | $n_{22}^{D}=1,5498$ |
| 40 | $2-C_2H_5$ | $6-C_4H_9(s)$ | H | 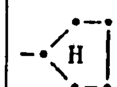 | $n_{22}^{D}=1,5464$ |

| Verbindung Nr. | $R_1$ | $R_2$ | $R_3$ | $R_4$ | physik. Daten |
|---|---|---|---|---|---|
| 41 | $2\text{-}S\text{-}C_3H_7(i)$ | 6-Cl | H | $-C_4H_9(t)$ | $n_{21}^{D}=1,5805$ |
| 42 | $2\text{-}C_3H_7(i)$ | $6\text{-}C_3H_7(i)$ | 4-O- 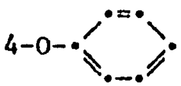 | $-(CH_2)_3\text{-}O\text{-}(CH_2)_3\text{-}CH_3$ | $n_{23}^{D}=1,5452$ |
| 43 | $2\text{-}C_3H_7(i)$ | $6\text{-}C_3H_7(i)$ | 4-O- 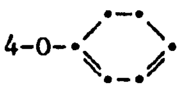 | $-(CH_2)_3\text{-}OCH_3$ | $n_{23}^{D}=1,5589$ |
| 44 | $2\text{-}C_3H_7(i)$ | $6\text{-}C_3H_7(i)$ | H | $-CH_2-$ 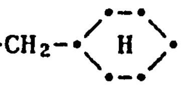 | $n_{21}^{D}=1,5408$ |
| 45 | $2\text{-}C_2H_5$ | $6\text{-}C_4H_9(s)$ | H | 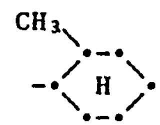 | $n_{21}^{D}=1,5402$ |
| 46 | $2\text{-}C_2H_5$ | $6\text{-}C_4H_9(s)$ | 4-O- 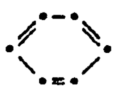 | 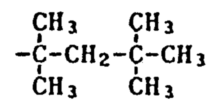 | $n_{21}^{D}=1,5482$ |
| 47 | $2\text{-}CH_3$ | $6\text{-}CH_3$ | 4-O- 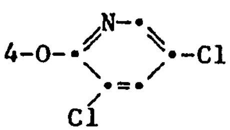 | 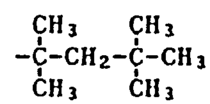 | $n_{21}^{D}=1,5730$ |
| 48 | $2\text{-}CH_3$ | $6\text{-}CH_3$ | 4-O- 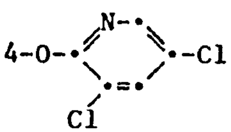 | 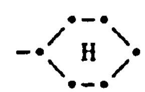 | Smp. 41-44°C |
| 49 | $2\text{-}C_3H_7(i)$ | H | 4-O- 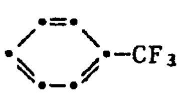 | $-CH(CH_3)\text{-}CH_2\text{-}OCH_3$ | $n_{21}^{D} = 1,5329$ |
| 50 | H | $2\text{-}C_3H_7(i)$ | 4-O- 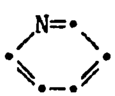 | $-CH_2\text{-}CH_2\text{-}CH_2\text{-}O\text{-}C_4H_9(n)$ | $n_{21}^{D} = 1,5548$ |
| 51 | H | $2\text{-}C_3H_7(i)$ | 4-O- 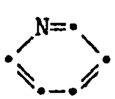 | $-C_4H_9(t)$ | Smp. 33-35°C |

| Verbindung Nr. | $R_1$ | $R_2$ | $R_3$ | $R_4$ | physik. Daten |
|---|---|---|---|---|---|
| 52 | H | $2-C_3H_7(i)$ | 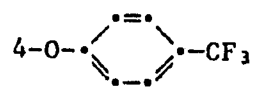 | 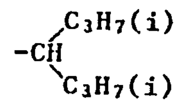 | $n_{21}^{D}=1,5249$ |
| 53 | $2-CH_3$ | $6-CH_3$ | 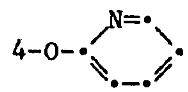 | $-C_4H_9(t)$ | $n_{21}^{D}=1.5768$ |
| 54 | $2-CH_3$ | $6-CH_3$ | 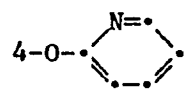 | $-CH(CH_3)-CH_2-OCH_3$ | $n_{21}^{D}=1,5792$ |
| 55 | $2-CH_3$ | $6-CH_3$ | 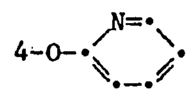 | 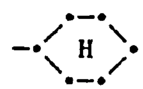 | $n_{21}^{D}=1,5989$ |
| 56 | $6-C_3H_7(i)$ | $2-C_3H_7(i)$ | H | 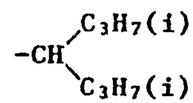 | $n_{21}^{D}=1,5200$ |
| 57 | $2-CH_3$ | $6-CH_3$ | 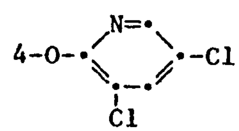 | $-C(CH_3)_2-C_2H_5$ | $n_{21}^{D}=1,5852$ |
| 58 | $2-CH_3$ | $6-CH_3$ | 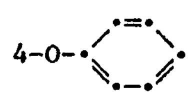 | 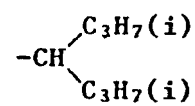 | $n_{21}^{D}=1,5662$ |
| 59 | $2-CH_3$ | $6-CH_3$ | 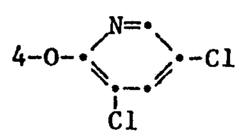 | 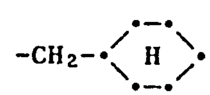 | $n_{21}^{D}=1,5950$ |
| 60 | $2-CH_3$ | $6-C_2H_5$ | H | $-C_3H_7(i)$ | $n_{23}^{D}=1,5396$ |
| 61 | $2-CH_3$ | $6-C_2H_5$ | H | $-C_4H_9(t)$ | $n_{23}^{D}=1,5296$ |

| Verbindung Nr. | $R_1$ | $R_2$ | $R_3$ | $R_4$ | physik. Daten |
|---|---|---|---|---|---|
| 62 | $2-C_3H_7(i)$ | $6-C_3H_7(i)$ | 4-O- 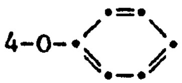 | $-(CH_2)_3-O-C_2H_5$ | $n_{23}^{D}=1,5527$ |
| 63 | $2-C_3H_7(i)$ | $6-C_3H_7(i)$ | 4-O- 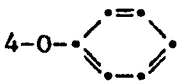 | $-(CH_2)_3-O-C_3H_7(i)$ | $n_{23}^{D}=1,5473$ |
| 64 | $2-C_3H_7(i)$ | $6-C_3H_7(i)$ | 4-O- 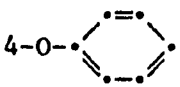 | $-C(CH_3)_2-CH_2-C(CH_3)_2-CH_3$ | $n_{23}^{D}=1,5466$ |
| 65 | $2-C_3H_7(i)$ | $6-C_3H_7(i)$ | 4-O- 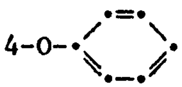 | - 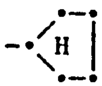 | $n_{23}^{D}=1,5680$ |
| 66 | $2-C_3H_7(i)$ | $6-C_3H_7(i)$ | 4-O- 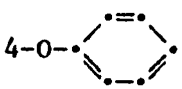 | - 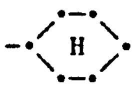 | $n_{23}^{D}=1,5702$ |
| 67 | $2-C_3H_7(i)$ | $6-C_3H_7(i)$ | 4-O- 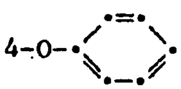 | $-CH_2-$ 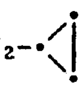 | $n_{23}^{D}=1,5740$ |
| 68 | $2-C_3H_7(i)$ | $6-C_3H_7(i)$ | 4-O- 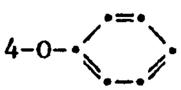 | $-CH_2-$ 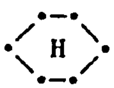 | $n_{23}^{D}=1,5663$ |
| 69 | $2-C_2H_5$ | $6-C_2H_5$ | H | $-C_3H_6(i)$ | $n_{23}^{D}=1,5358$ |
| 70 | $2-C_2H_5$ | $6-C_2H_5$ | H | $-C_4H_9(t)$ | $n_{23}^{D}=1,5294$ |
| 71 | $2-C_2H_5$ | $6-C_2H_5$ | 4-O- 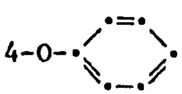 | $-C_3H_7(i)$ | $n_{25}^{D}=1,572$ |
| 72 | $2-C_2H_5$ | $6-C_2H_5$ | 4-O- 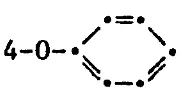 | $-C_4H_9(t)$ | $n_{23}^{D}=1,5670$ |

| Verbindung Nr. | $R_1$ | $R_2$ | $R_3$ | $R_4$ | physik.Daten |
|---|---|---|---|---|---|
| 73 | $2-C_3H_7(i)$ | $6-C_3H_7(i)$ | 4-O- 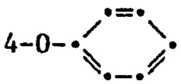 | $-C(CH_3)_2-C_2H_5$ | $n_{23}^{D}=1,5550$ |
| 74 | $2-C_2H_5$ | $6-C_2H_5$ | 4-O- 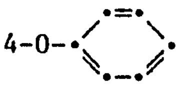 | 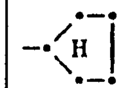 | $n_{23}^{D}=1,5844$ |
| 75 | $2-C_2H_5$ | $6-C_2H_5$ | 4-O- 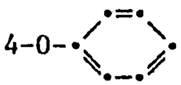 | $-C_4H_9(s)$ | $n_{23}^{D}=1,5703$ |
| 76 | $2-C_2H_5$ | $6-C_2H_5$ | 4-O- 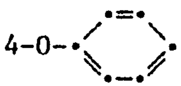 | $-C(CH_3)_2-C_2H_5$ | $n_{23}^{D}=1,5650$ |
| 77 | $2-CH_3$ | $6-CH_3$ | 4-O- 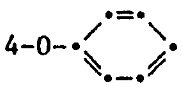 | 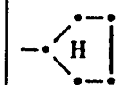 | $n_{21}^{D}=1,5951$ |
| 78 | $2-C_3H_7(i)$ | $6-C_3H_7(i)$ | 4-O- 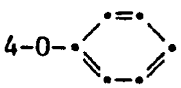 | 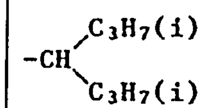 | $n_{21}^{D}=1,5510$ |
| 79 | $2-CH_3$ | $6-CH_3$ | 4-O- 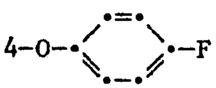 | $-C_4H_9(t)$ | $n_{24}^{D}=1,5618$ |
| 80 | $2-C_2H_5$ | $6-C_2H_5$ | 4-O- 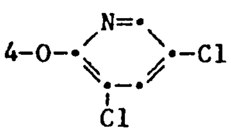 | $-C_4H_9(t)$ | $n_{24}^{D}=1,5770$ |
| 81 | $2-CH_3$ | $6-CH_3$ | 4-O- 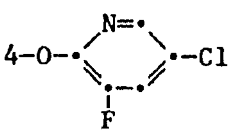 | $-C_4H_9(t)$ | $n_{24}^{D}=1,5720$ |

| Verbindung Nr. | $R_1$ | $R_2$ | $R_3$ | $R_4$ | physik. Daten |
|---|---|---|---|---|---|
| 82 | $2-CH_3$ | $6-C_2H_5$ | 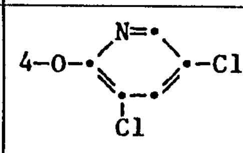 | $-C_4H_9(t)$ | $n_{24}^{D}=1,5820$ |
| 83 | $2-CH_3$ | $6-CH_3$ | 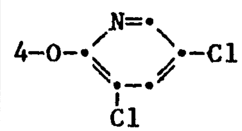 | $-C_3H_7(i)$ | $n_{24}^{D}=1,5983$ |
| 84 | $2-CH_3$ | $6-CH_3$ | 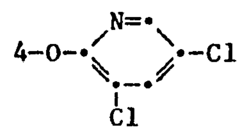 | 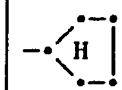 | Smp. 65-68°C |
| 85 | $2-CH_3$ | $6-CH_3$ | 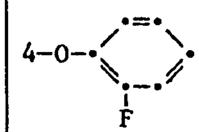 | $-C_4H_9(t)$ | $n_{24}^{D}=1,5640$ |

| Verbindung Nr. | $R_1$ | $R_2$ | $R_3$ | $R_4$ |
|---|---|---|---|---|
| 86 | $2-C_2H_5$ | $6-C_4H_9(s)$ | 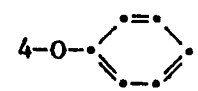 | 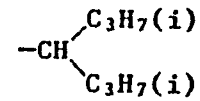 |
| 87 | $2-CH_3$ | $6-CH_3$ | 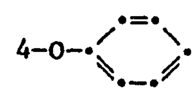 | 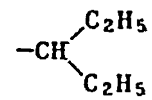 |
| 88 | $2-CH_3$ | $6-CH_3$ | 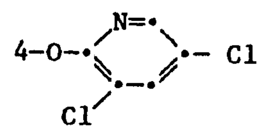 | 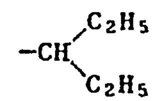 |
| 89 | $2-C_3H_7(i)$ | $6-C_3H_7(i)$ | 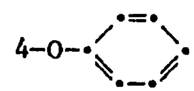 | 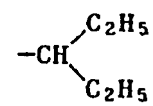 |
| 90 | $2-C_2H_5$ | $6-C_4H_9(s)$ | 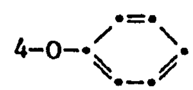 | 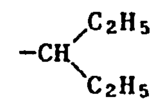 |
| 91 | $2-CH_3$ | $6-CH_3$ | 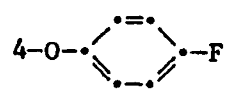 | 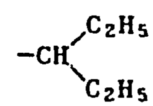 |
| 92 | $2-C_3H_7(i)$ | $6-C_3H_7(i)$ | 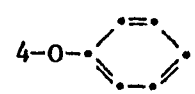 | 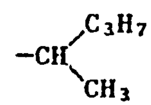 |

| Verbindung Nr. | $R_1$ | $R_2$ | $R_3$ | $R_4$ |
|---|---|---|---|---|
| 93 | $2-C_3H_7(i)$ | $6-C_3H_7(i)$ | H | 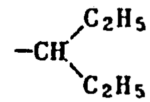 |
| 94 | $2-CH_3$ | $6-CH_3$ | 4-O-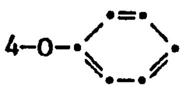 | 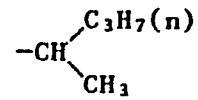 |
| 95 | $2-C_3H_7(i)$ | $6-C_3H_7(i)$ | 4-O-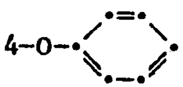 | 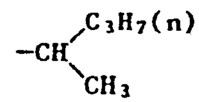 |
| 96 | $2-CH_3$ | $6-CH_3$ | 4-O-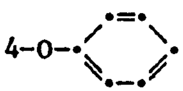 | 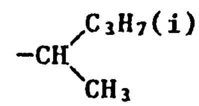 |
| 97 | $2-CH_3$ | $6-CH_3$ | 4-O-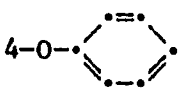 | $-(CH_2)_5CH_3$ |
| 98 | $2-CH_3$ | $6-CH_3$ | 4-O-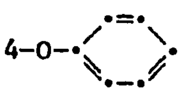 | $-(CH_2)_{11}CH_3$ |
| 99 | $2-C_2H_5$ | $6-C_2H_5$ | H | $-C_3H_7(i)$ |
| 100 | $2-C_2H_5$ | $6-C_2H_5$ | 4-O-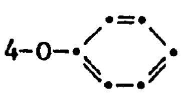 | $-C_3H_7(i)$ |

| Verbindung Nr. | $R_2$ | $R_3$ | $R_4$ | $R_5$ |
|---|---|---|---|---|
| 101 | $2-C_2H_5$ | $6-C_2H_5$ | 4-O- 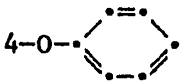 | $-C_4H_9(t)$ |
| 102 | $2-C_3H_7(i)$ | $6-C_3H_7(i)$ | 4-O- 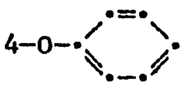 | $-C(CH_3)_2-C_2H_5$ |
| 103 | $2-C_2H_5$ | $6-C_2H_5$ | 4-O- 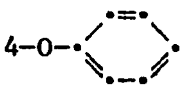 | 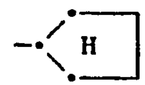 |
| 104 | $2-C_2H_5$ | $6-C_2H_5$ | 4-O- 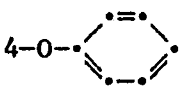 | $-C_4H_9(s)$ |
| 105 | $2-C_2H_5$ | $6-C_2H_5$ | 4-O- 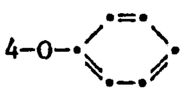 | $-C(CH_3)_2-C_2H_5$ |
| 106 | $2-C_2H_5$ | $6-C_2H_5$ | 4-O- 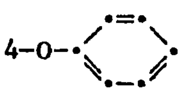 | $-CH(CH_3)-CH_2-O-CH_3$ |
| 107 | $2-C_2H_5$ | $6-C_4H_9(s)$ | 4-O- 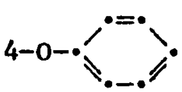 | $-C_4H_9(s)$ |

| Verbindung Nr. | $R_1$ | $R_2$ | $R_3$ | $R_4$ |
|---|---|---|---|---|
| 108 | $2-C_4H_9(s)$ | $6-C_2H_5$ | 4-O- 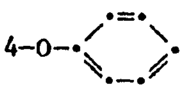 | $-CH(CH_3)-CH_2-O-CH_3$ |
| 109 | $2-C_2H_5$ | $6-C_4H_9(s)$ | 4-O- 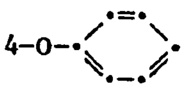 | 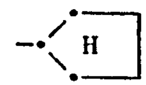 |
| 110 | $2-C_2H_5$ | $6-C_4H_9(s)$ | 4-O- 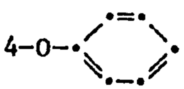 | $-C(CH_3)_2-C_2H_5$ |
| 111 | $2-C_2H_5$ | $6-C_3H_7(i)$ | 4-O- 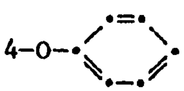 | $-C_4H_9(s)$ |
| 112 | $2-C_2H_5$ | $6-C_3H_7(i)$ | 4-O- 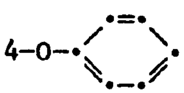 | $-C(CH_3)_2-C_2H_5$ |
| 113 | $2-C_2H_5$ | $6-C_3H_7(i)$ | 4-O- 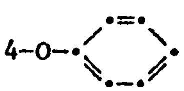 | $-CH(CH_3)-CH_2-O-CH_3$ |

| Verbindung Nr. | $R_2$ | $R_3$ | $R_4$ | $R_5$ |
|---|---|---|---|---|
| 114 | $2-C_2H_5$ | $6-C_3H_7(i)$ | 4-O- 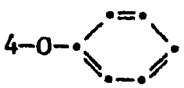 | 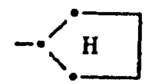 |
| 115 | $2-C_3H_7(i)$ | $6-C_3H_7(i)$ | 4-O- 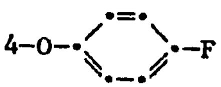 -F | $-C_4H_9(t)$ |
| 116 | $2-C_3H_7(i)$ | $6-C_3H_7(i)$ | 4-O- 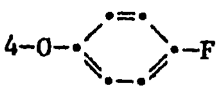 -F | 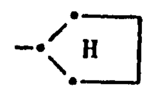 |

Beispiel 2

Formulierungen für flüssige Wirkstoffe der Formel I gemäss Beispiel 1 oder Kombinationen dieser Wirkstoffe mit anderen Insektiziden oder Akariziden (% = Gewichtsprozent):

| 2.1. Emulsions-Konzentrate | a) | b) | c) |
|---|---|---|---|
| Wirkstoff resp. Wirkstoffkombination | 25% | 40% | 50% |
| Ca-Dodecylbenzolsulfonat | 5% | 8% | 6% |
| Ricinusölpolyäthylenglykoläther (36 Mol AeO) | 5% | — | — |
| Tributylphenolpolyäthylenglykoläther (30 Mol AeO) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff resp. Wirkstoffkombination | 80% | 10% | 5% | 95% |
| Aethylenglykolmonomethyläther | 20% | — | — | — |
| Polyäthylenglykol MG 400 | — | 70% | — | — |
| N-Methyl-2-pyrrolidon | — | 20% | — | — |
| Epoxidiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

| 2.3. Granulate | a) | b) |
|---|---|---|
| Wirkstoff resp. Wirkstoffkombination | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff resp. Wirkstoffkombination | 2% | 5% |
| Hochdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemittel.

Formulierungen für feste Wirkstoffe der Formel I gemäss Beispiel 1 oder Kombinationen dieser Wirkstoffe mit andern Insektiziden oder Akariziden (% = Gewichtsprozent):

| 2.5. Spritzpulver | a) | b) | c) |
|---|---|---|---|
| Wirkstoff oder Wirkstoffkombination | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykoläther (7—8 Mol AeO) | — | 2% | — |
| Hochdisperse Kieselsäure | 5% | 10% | 10% |
| Kaolin | 62% | 27% | — |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

| 2.6 Emulsions-Konzentrat | |
|---|---|
| Wirkstoff oder Wirkstoffkombination | 10% |
| Octylphenolpolyäthylenglykoläther (4—5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff oder Wirkstoffkombination | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

| 2.8. Extruder-Granulat | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

2.9. Umhüllungs-Granulat

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

2.10. Suspensions-Konzentrat

| | |
|---|---|
| Wirkstoff oder Wirkstoffkombination | 40% |
| Aethylenglykol | 10% |
| Nonylphenolpolyäthylenglykoläther (15 Mol AeO) | 5% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3

Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpfen Fliegen bestimmt.

Verbindungen der Formel I gemäss Beispiel 1 zeigen gute Wirkung im obigen test.

Beispiel 4

Wirkung gegen Lucilia sericata

Zu 9 ml eines Zuchtmediums wird 1 ml einer 0,5% Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 5

Wirkung gegen Aëdes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach

Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss Beispiel 1 ziegen gute Wirkung im obigen Test.

Beispiel 6

Insektizide Frassgift-Wirkung

Baumwollpflanzen (ca. 20 cm hoch) werden mit wässrigen Wirkstoffemulsionen (erhalten aus einem 10%igen emulgierbaren Konzentrat) besprüht, wobei die Wirkstoffemulsionen 100 bis 400 ppm der zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24°C und 60% relativer Luftfeuchtigkeit durchgeführt. In Abständen von jeweils 24 Stunden werden Mortalität sowie Entwicklungs- und Häutungsstörungen der angesetzten Larven bestimmt.

Die Verbindungen Nr. 24 und 25 zeigen bei 100 ppm pzw. 400 ppm 80—100% Wirkung (Mortalität) gegen Heliothis-Larven.

Erfindungsgemässe Verbindungen gemäss Beispiel 1 zeigen in folgenden Konzentrationen gegen Larven von Spodoptera littoralis 80—100%ige Wirksamkeit (Mortalität):

| Verbindung Nr. | Wirkstoffkonzentration |
|---|---|
| 25 | 200 ppm |
| 23, 62, 63, 65, 67 und 68 | 400 ppm |

Beispiel 7

Wirkung auf Spodoptera littoralis und Heliothis virescens (Larven und Eier)

Es werden drei in Töpfen gezogene Baumwollpflanzen von ca. 15—20 cm Höhe mit einer sprühfähigen flüssigen Zubereitung des zu prüfenden Wirkstoffes in einer Konzentration von 800 ppm behandelt. Nach Antrocknen des Sprühbelages werden die eingetopften Pflanzen in ein Blechgefäss von etwa 20 Litern Inhalt gestgellt, das mit einer Glasplatte abgedeckt wird. Die Feuchtigkeit im Inneren des abgedeckten Gefässes wird so reguliert, dass sich kein Kondenswasser bilden kann. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens (dazu werden je 2 Blätter einer der Baumvoll-Pflanzen in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis werden zu den eingeschlossenen Blättern gegeben).

Nach 4 und 5 Tagen erfolgt die Auswertung gegenüber unbehandelten Kontrollen unter Berücksichtigung folgender Kriterien:

a) Anzahl der noch lebenden Larven,

b) larvale Entwicklungs- und Häutungsstörungen,

c) Frassschaden (Schabfrass und Lochfrass),

d) Schlupfrate (Anzahl der aus den Eiern geschlüpften Larven).

Verbindungen der formel I gemäss Beispiel 1 zeigen gute Gesamt-Wirksamkeit in obigem Test.

Beispiel 8

Ovizide Wirkung auf Spodoptera littoralis

Auf Filterpapier abgelegte Eier von Spodoptera littoralis werden aus dem Papier ausgeschnitten und in eine Lösung von 400 ppm des Wirkstoffes in einem Aceton-Wasser-Gemisch (1:1) getaucht. Die so behandelten Eiablagen werden dann aus diesem Gemisch herausgenommen und bei 28°C und 60% relativer Feuchtigkeit in Kunststoffschalen deponiert.

Nach 5 tagen wird die Schlupfrate, d.h. die Anzahl Larven, die sich aus den behandelten Eiern entwickelt haben, bestimmt.

Die Verbindungen Nr. 60, 61, 63, 64 und 69 gemäss Beispiel 1 zeigen 80—100% Wirkung (Mortalität) im obigen Test.

Beispiel 9

Ovizide Wirkung auf Laspeyresia pomonella

Abgelegte Eier von Laspeyresia pomonella, die nicht älter als 24 Stunden sind, werden auf Filterpapier für 1 Minute in acetonisch-wässrige Lösungen enthaltend bis zu 400 ppm des zu prüfenden Wirkstoffes eingetaucht. Nach dem Antrocknen der Lösung werden die Eier in Petrischalen ausgelegt und bei einer

Temperatur von 28°C belassen. Nach 6 Tagen wird der prozentuale Schlupf aus den behandelten Eiern bewertet.

Verbindung Nr. 5 gemäss Beispiel 1 zeigt schon bei 200 ppm 100% Wirkung (Mortalität) in obigem Test.

Beispiel 10

Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 400 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antiefeedant-Wirkung gegenüber unbehandelten Kontrollansätzen.

Die Verbindungen Nr. 9, 60 und 61 zeigen 80—100% Wirkung (Mortalität) in diesem Test.

Beispiel 11

Wirkung gegen pflanzenschädigende Akariden: Tetranychus urticae (OP-sensible) und Tetranychus cinnabarinus (OP-tolerant).

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt (Die Toleranz bezieht sich auf die Verträglichkeit gegenüber Diazinon).

Die so behandelten infestierten Pflanzen werden mit einer Versuchslösung enthaltend 0,75 bis 400 ppm der jeweils zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendet pro Konzentration und pro Testspezies eine Pflanze. Während des Versuchsverlaufs stehen die Pflanzen in Gewächshauskabinen bei 25°C.

Verbindungen der Formel I gemäss Beispiel 1 zeigen in diesem Versuch gute Wirkung gegen Tetranychus urticae.

Erfindungsgemässe Verbindungen zeigen in folgenden Konzentrationen gegen Tetranychus cinnabarinus 80—100%ige Wirksamkeit (Mortalität):

| Verbindung Nr. | Wirkstoffkonzentration |
|---|---|
| 1 und 42 | 0,75 ppm |
| 19, 63, 65 | 12,5 ppm |

Beispiel 12

Insektizide Kontaktwirkung: Myzus persicae

Etwa 4 cm hohe, in Wasser angezogene Erbesenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 12,5, 50, 200 und 400 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20—22°C und 60% relativer Luftfeuchtigkeit durchgeführt.

Die Verbindung Nr. 7 zeigt in diesem Test schon bei einer Konzentration von 12,5 ppm eine Wirkung von 80—100% (Mortalität). Die gleiche Wirkung zeigen die Verbindungen Nr. 25 und 26 bei 50 ppm, die Verbindungen Nr. 23, 25 und die Verbindung Nr. 70 bei 400 ppm.

Beispiel 13

Insektizide Kontaktwirkung: Aphis craccivora

In Töpfen angezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 12,5, 50, 100, 200 und 400 ppm der zu prüfenden Verbindung bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Erfindungsgemässe Verbindungen zeigen in diesem Test bei den nachstehend aufgeführten Konzentrationen eine Wirkung von 80—10% (Mortalität):

| Verbindung Nr. | Wirkstoffkonzentration |
|---|---|
| 6, 36, 40 | 12,5 ppm |
| 23, 25, 26, 27, 33 35 und 37 | 50 ppm |
| 79, 65, 66 u. 70 | 100 ppm |
| 24, 39, 44 und 45 | 200 ppm |
| 60, 62, 67 und 68 | 400 ppm |

Beispiel 14

Wirkung gegen Laodelphax striatellus und Nilaparvata lugens (Nymphen)

Der Test wird an wachsenden Pflanzen durchgeführt. Dazu werden jeweils 4 Reispflanzen (Dicke des Stengels ca. 8 mm) mit einer Höhe von ca. 20 cm in Töpfe (Durchmesser von 8 cm) eingeflanzt.

Die Pflanzen werden auf einem Drehteller mit jeweils 100 ml von acetonischen Lösungen enthaltend 50 bis 400 ppm des zu prüfenden Wirkstoffs besprüht. Nach dem Antrocknen des Spriztbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an den behandelten Pflanzen gehalten. Die Auswertung auf %-Mortalität erfolgt 1, 4 und 8 Tage nach der Behandlung.

Erfindungsgemässe Verbindungen zeigen in folgenden Konzentrationen gegen Nymphen von Nilaparvata lugens nach 8 Tagen 80—100%ige Wirksamkeit (Mortalität):

| Verbindung Nr. | Wirkstoffkonzentration |
|---|---|
| 36 | 12,5 ppm |
| 7, 9, 33 und 34 | 50 ppm |
| 6, 13 und 65 | 100 ppm |
| 23, 26 und 45 | 200 ppm |
| 25, 26, 38, 47, 48, 67, 68, 70, 71, 72 und 74 | |
| 24, 25, 37, 46, 47, 63, 64, 66, 67, 68 und 70 | 400 ppm |

Mit den Verbindungen der Formel I gemäss Beispiel 1 kann gegen Nymphen von Laodelphax striatellus ebenfalls gute Wirksamkeit erzielt werden.

Beispiel 14

Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in steigenden Konzentrationen von 3 ppm bis 200 ppm enthalten. Dann werden Plastikbecher, die einen oberen Durchmeser von ca. 10 cm haben, mit der so behandelten Erde teilweise gefüllt. Pro Becker werden zehn larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Anstatz wird die in den Bechern enthaltene Erde abgesiebt und die zurückbleibenden Larven werden hinsichtlich ihrer Mortalität geprüft.

Erfindungsgemässe Verbindungen zeigen in diesem Test bei den nachstehend aufgeführten Konzentrationen eine Wirkung von 80—100% (Mortalität):

| Verbindung Nr. | Wirkstoffkonzentration |
|---|---|
| 23 und 36 | 3 ppm |
| 25, 35, 37 und 45 | 12,5 ppm |
| 26, 29, 34, 44<br>27, 30, 35 und 45 | 50 ppm |
| 53, 61 und 69 | 100 ppm |
| 9 | 200 ppm |
| 60, 63, 67 und 70 | 400 ppm |

Beispiel 16

Wirkung gegen Panonychus ulmi (OP und Carbamat resistent)

Eingetopfte Apfelsämlinge mit etwa 20—30 Blättern werden mit jeweils 60 adulten Weibchen von Panonychus ulmi besiedelt. Nach sieben Tagen werden die infestierten Pflanzen mit einer wässrigen Emulsion enthaltend 0,75 ppm der zu prüfenden Verbindung bis zur Tropfnässe besprüht. Die behandelten Pflanzen werden dann während weiteren 14 Tagen bei 25°C und etwa 50% relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Die Verbindungen Nr. 1 und 19 gemäss Beispiel 1 zeigen in diesem Test eine 80—100%ige Wirkung (Mortalität).

**Patentansprüche**

1. Verbindung der Formel I

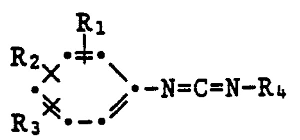

(I),

worin

$R_1$ Wasserstoff, Halogen, $C_1$—$C_4$-Alkyl, Methoxy, Aethoxy, Trifluormethoxy oder $C_1$—$C_3$-Alkylthio;

$R_2$ Wasserstoff, Halogen, $C_1$—$C_4$-Alkyl, Methoxy oder Aethoxy,

$R_3$ Wasserstoff, $C_1$—$C_4$-Alkyl, Halogen, Phenoxy, Phenylthio, ein- oder zweifach mit Halogen und/oder Trifluormethyl substituiertes Phenoxy, Pyridyloxy oder ein- oder zweifach mit Halogen oder Trifluormethyl substituiertes Pyridyloxy; und

$R_4$ $C_1$—$C_8$-Alkyl, Alkoxyalkyl mit insgesamt 2 bis 7 C-Atomen, $C_3$—$C_5$-Cycloalkyl, $C_3$—$C_6$-Cycloalkylmethyl oder Methyl-($C_3$—$C_6$)-Cycloalkyl;

bedeuten, wobei mindestens zwei der Reste $R_1$, $R_2$ oder $R_3$ eine von Wasserstoff abweichende Bedeutung besitzen, $R_3$ in 4-Stellung und $R_1$ und $R_2$ unabhängig voneinander in 2- bzw. 6-Stellung am Phenylrest stehen.

2. Verbindung gemäss Anspruch 1, dadurch gekennzeichnet, dass

$R_1$ Wasserstoff, Fluor, Chlor, $C_2$—$C_4$-Alkyl oder Methoxy;

$R_2$ Wasserstoff, Chlor, $C_3$—$C_4$-Alkyl oder Methoxy;

$R_3$ Methyl, Aethyl, Isopropyl, Chlor, Phenoxy, ein- oder zweifach mit Chlor und/oder Trifluormethyl substituiertes Phenoxy, Pyridyloxy oder ein- oder zweifach mit Chlor und/oder Trifluormethyl substituiertes Pyridyloxy; und

$R_4$ $C_3$—$C_8$-Alkyl, $C_1$—$C_4$-Alkoxy-($C_1$—$C_3$)-Alkyl oder $C_6$—$C_6$-Cycloalkyl bedeuten.

3. Verbindung gemäss Anspruch 1 oder 2, dadurch gekennzeichnet, dass $R_4$ Isopropyl oder tert. Butyl bedeutet.

4. Verbindung gemäss Anspruch 3 der Formel

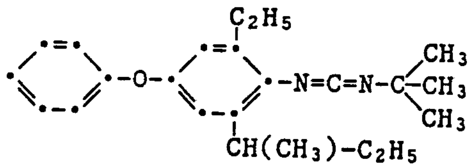

5. Verbindung gemäss Anspruch 2 der Formel

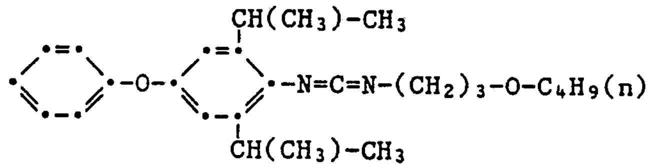

6. Verbindung gemäss Anspruch 2 der Formel

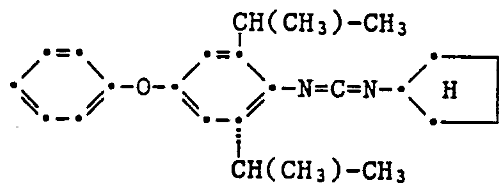

7. Verbindung gemäss Anspruch 3 der Formel

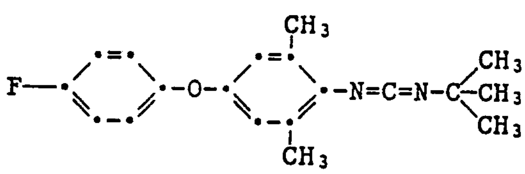

8. Verbindung gemäss Anspruch 3 der Formel

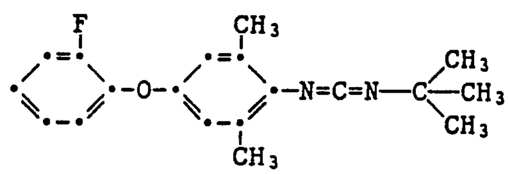

9. Verbindung gemäss Anspruch 3 der Formel

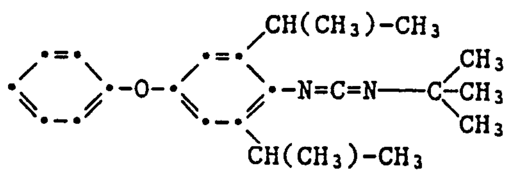

10. Verbindung gemäss Anspruch 2 der Formel

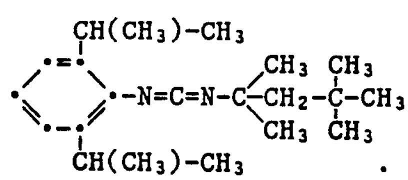

11. Verbindung gemäss Anspruch 3 der Formel

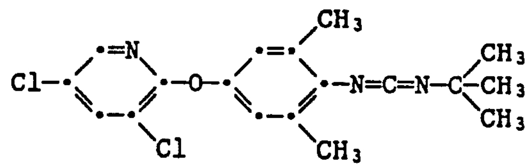

12. Verbindung gemäss Anspruch 3 der Formel

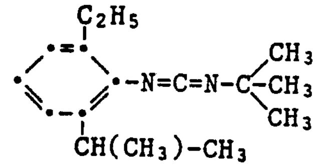

13. Verbindung gemäss Anspruch 3 der Formel

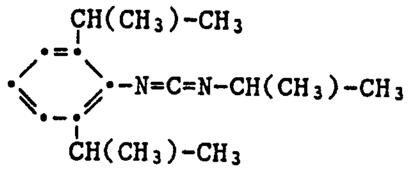

14. Verfahren zur Herstellung einer Verbindung der Formel I gemäss einem der Ansprüche 1 bis 13, dadurch gekennzeichnet, dass man aus einer Verbindung der Formel II

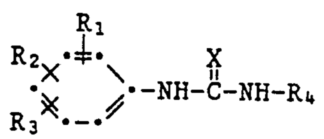

(II),

worin $R_1$ bis $R_4$ die unter Anspruch 1 bis 3 angegebenen Bedeutungen haben und X Sauerstoff oder Schwefel bedeutet, Wasser bzw. Schwefelwasserstoff abspaltet.

15. Schädlingsbekämpfungsmittel, welches als aktive Komponente eine Verbindung gemäss einem der Ansprüche 1 bis 13 zusammen mit geeigneten Trägern und/oder anderen Zuschlagstoffen enthält.

16. Verwendung einer Verbindung gemäss einem der Ansprüche 1 bis 13 zur Bekämpfung von Insekten und Vertretern der Ordnung Akarina an Tieren und Pflanzen.

17. Verwendung gemäss Anspruch 16 zur Bekämpfung von pflanzenschädigenden Insekten.

**Revendications**

1. Composé de formule I

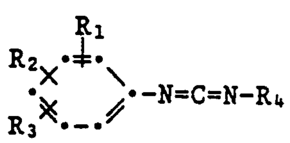

(I),

dans laquelle

$R_1$ représente l'hydrogène, un halogène, un groupe alkyle en C 1—C 4, méthoxy, éthoxy, trifluorométhoxy ou alkylthio en C 1—C 3,

$R_2$ représente l'hydrogène, un halogène, un groupe alkyle en C 1—C 4, méthoxy ou éthoxy,

$R_3$ représente l'hydrogène, un groupe alkyle en C 1—C 4, un halogène, un groupe phénoxy, phénylthio, un groupe phénoxy mono- ou di-substitué par des halogènes et/ou des groupes trifluorométhyle, un groupe pyridyloxy ou un groupe pyridyloxy mono- ou di-substitué par des halogènes ou des groupes trifluorométhyle, et

$R_4$ représente un groupe alkyle en C 1—C 8, alcoxyalkyle contenant au total 2 à 7 atomes de carbone, cycloalkyle en C 3—C 5, (cycloalkyle en C 3—C 6)-méthyle ou méthylcycloalkyle en C 3—C 6;

deux au moins des symboles $R_1$, $R_2$ et $R_3$ ayant des significations autres que l'hydrogène, $R_3$ se trouve en position 4 et $R_1$ et $R_2$ occupent, indépendamment l'un de l'autre, les positions respectives 2 et 6 du noyau phényle.

2. Composé selon la revendication 1, caractérisé en ce que:

$R_1$ représente l'hydrogène, le fluor, le chlore, un groupe alkyle en C 2—C 4 ou méthoxy;

$R_2$ représente l'hydrogène, le chlore, un groupe alkyle en C 3—C 4 ou méthoxy;

$R_3$ représente un groupe méthyle, éthyle, isopropyle, le chlore, un groupe phénoxy, un groupe phénoxy mono- ou di-substitué par le chlore et/ou des groupes trifluorométhyle, un groupe pyridyloxy ou un groupe pyridyloxy mono- ou di-substitué par le chlore et/ou des groupes trifluorométhyle; et

$R_4$ représente un groupe alkyle en C 3—C 8, (alcoxy en C 1—C 4)-alkyle en C 1—C 3 ou cycloalkyle en C 3—C 6.

3. Composé selon la revendication 1 ou 2, caractérisé en ce que $R_4$ représente un groupe cyclopropyle ou tert-butyle.

4. Composé selon la revendication 3, de formule

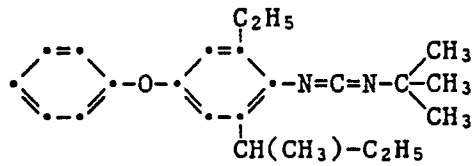

5. Composé selon la revendication 2, de formule

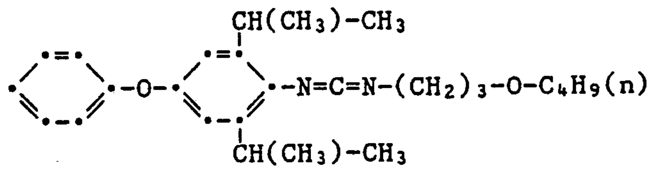

6. Composé selon la revendication 2, de formule

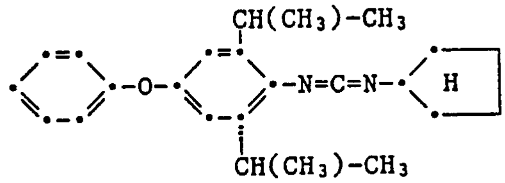

7. Composé selon la revendication 3, de formule

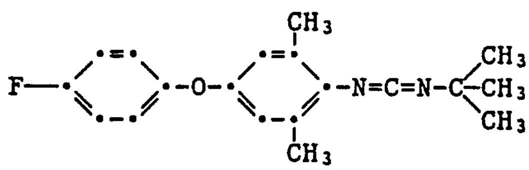

8. Composé selon la revendication 3, de formule

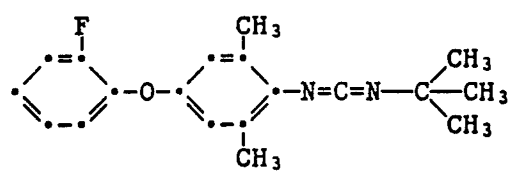

9. Composé selon la revendication 3, de formule

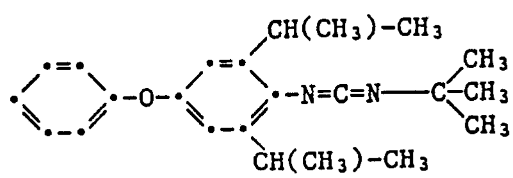

10. Composé selon la revendication 2, de formule

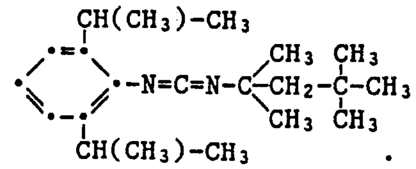

11. Composé selon la revendication 3, de formule

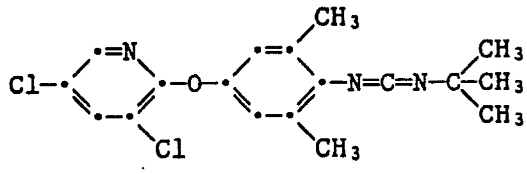

12. Composé selon la revendication 3, de formule

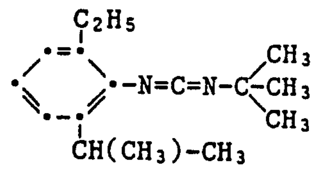

13. Composé selon la revendication 3, de formule

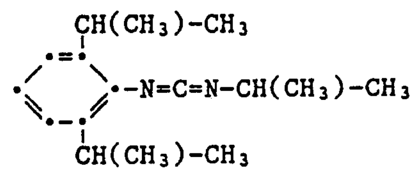

14. Procédé de préparation d'un composé de formule I selon l'une des revendications 1 à 13, caractérisé en ce que, partant d'un composé de formule II

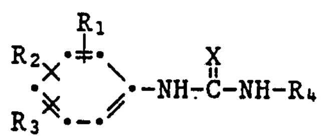

(II),

dans laquelle $R_1$ à $R_4$ ont les significations indiquées dans les revendications 1 à 3, et X représente l'oxygène ou le soufre, on élimine de l'eau ou du sulfure d'hydrogène respectivement.

15. Produit pesticide contenant en tant que composant actif un composé l'une des revendications 1 à 13 avec des véhicules et/ou d'autres additifs appropriés.

16. Utilisation d'un composé selon l'une des revendications 1 à 13, pour la lutte contre les insectes et les représentants de l'ordre des acariens sur les animaux et les végétaux.

17. Utilisation selon la revendication 16, pour la lutte contre les insectes nuisibles pour les végétaux.

**Claims**

1. A compound of formula I

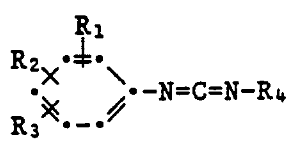

(I),

wherein

$R_1$ is hydrogen, halogen, $C_1$—$C_4$alkyl, methoxy, ethoxy, trifluoromethoxy or $C_1$—$C_3$alkylthio;

$R_2$ is hydrogen, halogen, $C_1$—$C_4$alkyl, methoxy or ethoxy;

$R_3$ is hydrogen, $C_1$—$C_4$alkyl, halogen, phenoxy, phenylthio, phenoxy which is mono- or disubstituted by halogen and/or trifluoromethyl, or is pyridyloxy or pyridyloxy which is substituted by one or two members selected from the group consisting of halogen and trifluoromethyl; and

$R_4$ is $C_1$—$C_8$alkyl, alkoxyalkyl containing a total of 2 to 7 carbon atoms, $C_3$—$C_5$cycloalkyl, $C_3$—$C_6$cycloalkylmethyl, methyl($C_3$—$C_6$)cycloalkyl, with the proviso that at least two of the radicals $R_1$, $R_2$ or $R_3$ have a meaning different from hydrogen, $R_3$ is in the 4-position and $R_1$ and $R_2$ independently of each other are in the 2- and 6-position of the phenyl ring.

2. A compound according to claim 1, wherein
$R_1$ is hydrogen, fluoro, chloro, $C_2$—$C_4$alkyl or methoxy;
$R_2$ is hydrogen, chloro, $C_3$—$C_4$alkyl or methoxy;
$R_3$ is methyl, ethyl, isopropyl, chloro, phenoxy, phenoxy which is mono- or disubstituted by chloro and/or trifluoromethyl, or is pyridyloxy or pyridyloxy which is mono- or disubstituted by chloro and/or trifluoromethyl; and
$R_4$ is $C_3$—$C_8$alkyl, $C_1$—$C_4$alkoxy($C_1$—$C_3$)alkyl or $C_3$—$C_6$cycloalkyl.

3. A compound according to either claim 1 or claim 2, wherein $R_4$ is isopropyl or tert-butyl.

4. A compound according to claim 3 of formula

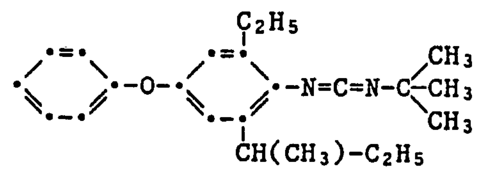

5. A compound according to claim 2 of formula

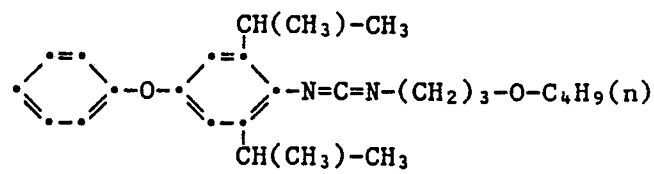

6. A compound according to claim 2 of formula

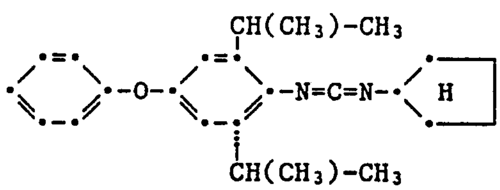

7. A compound according to claim 3 of formula

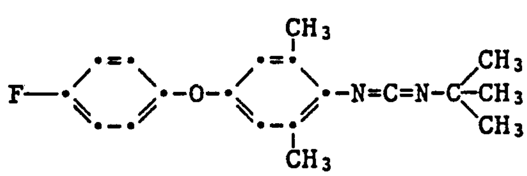

8. A compound according to claim 3 of formula

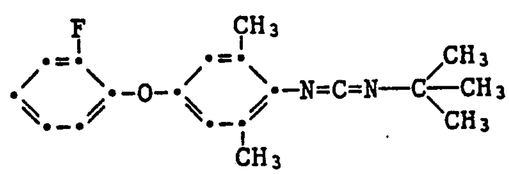

9. A compound according to claim 3 of formula

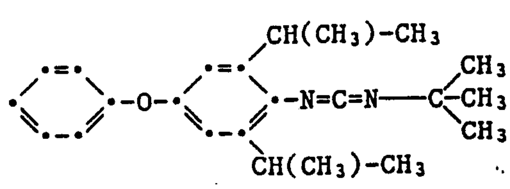

10. A compound according to claim 2 of formula

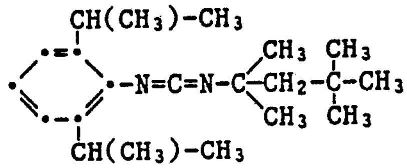

11. A compound according to claim 3 of formula

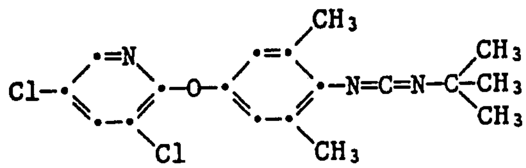

12. A compound according to claim 3 of formula

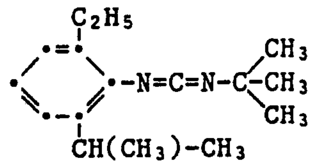

13. A compound according to claim 3 of formula

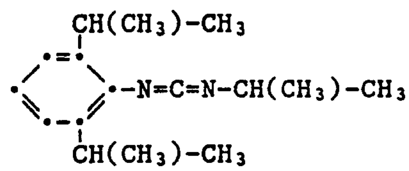

14. A process for the preparation of a compound of formula I according to any one of claims 1 to 13, which process comprises removing water or hydrogen sulfide respectively from a compound of formula II

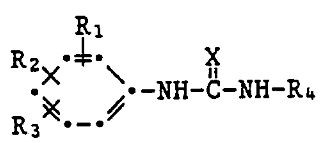 (II),

wherein $R_1$ to $R_4$ are as defined in any claims 1 to 3 and X is oxygen or sulfur.

15. A pesticidal composition which contains as active component a compound according to any one of claims 1 to 13, together with suitable carriers and/or other adjuvants.

16. Use of a compound according to any one of claims 1 to 13 for controlling insects and representatives of the order Acarina on animals and plants.

17. Use according to claim 16 for controlling plant-destructive insects.